# EUROPEAN PATENT SPECIFICATION

(11) **EP 1 969 135 B1**
(45) Date of publication and mention of the grant of the patent: **21.06.2017**
(21) Application number: 06847955.9
(22) Date of filing: 21.12.2006
(51) Int. Cl.: C12Q 1/00, G01N 27/30, G01N 33/487

(54) **PROCESS OF MAKING ELECTRODES FOR TEST SENSORS**
VERFAHREN ZUR HERSTELLUNG VON ELEKTRODEN FÜR TESTSENSOREN
PROCEDE DE FABRICATION D'ELECTRODES POUR CAPTEURS TESTS

(30) Priority: 27.12.2005 US 754146 P
(43) Date of publication of application: 17.09.2008
(73) Proprietor: Ascensia Diabetes Care Holdings AG, 4052 Basel (CH)
(72) Inventor: EDELBROCK, Andrew, J., Granger, Indiana 46530 (US); CHARLTON, Steven, C., Osceola, Indiana 46561 (US)
(74) Representative: Cohausz & Florack
(86) International application number: PCT/US2006/048876
(87) International publication number: WO 2007/075936

(56) References cited:
- US-A- 5 437 999
- US-A- 5 863 400
- US-A1- 2004 251 132
- US-A1- 2005 098 433
- US-A1- 2005 114 062

## Description

### FIELD OF THE INVENTION

The present invention generally relates to a process of making electrodes for test sensors. More specifically, the process is directed to making electrodes for test sensors, in which the test sensors are adapted to be used in instruments or meters that determine the concentration of an analyte (e.g., glucose) in a fluid.

### BACKGROUND OF THE INVENTION

The quantitative determination of analytes in body fluids is of great importance in the diagnoses and maintenance of certain physiological abnormalities. For example, lactate, cholesterol and bilirubin should be monitored in certain individuals. In particular, it is important that diabetic individuals frequently check the glucose level in their body fluids to regulate the glucose intake in their diets. The results of such tests can be used to determine what, if any, insulin or other medication needs to be administered. In one type of blood-glucose testing system, sensors are used to test a sample of blood.

A test sensor contains biosensing or reagent material that reacts with blood glucose. The testing end of the sensor is adapted to be placed into the fluid being tested, for example, blood that has accumulated on a person's finger after the finger has been pricked. The fluid is drawn into a capillary channel that extends in the sensor from the testing end to the reagent material by capillary action so that a sufficient amount of fluid to be tested is drawn into the sensor. The fluid then chemically reacts with the reagent material in the sensor resulting in an electrical signal indicative of the glucose level in the fluid being tested. This signal is supplied to the meter via contact areas located near the rear or contact end of the sensor and becomes the measured output.

One method of currently forming electrodes and leads on a test sensor is by laminating a substrate with a metal foil followed by a subtractive cutting/ablation process to define the electrodes and leads. Another method currently being used includes sputtering a metal onto the substrate and subsequently removing the metal by a subtractive cutting/ablation process to define the electrodes and leads. These existing processes tend to be more costly than necessary because a portion of the metallic material is removed from the substrate and, thus, is not present in finalized test sensor. Additionally, these existing metallic deposition processes themselves can be costly.

It would be desirable to provide a method for forming the electrodes and leads on the test sensor that is more cost-effective than existing processes.

### SUMMARY OF THE INVENTION

According to one method, a plurality of electrodes is formed on a test sensor. The test sensor assists in determining the concentration of an analyte. A substrate is provided. At least one aperture is formed through the substrate. Catalytic ink or catalytic polymeric solution is applied in a pattern on two sides of the substrate and on a surface adjacent to and forming the at least one aperture. The catalytic ink or catalytic polymeric solution on the two sides assists in defining the plurality of electrodes on the test sensor. After applying the catalytic ink or catalytic polymeric solution, the substrate is electrolessly plated to form the plurality of the electrodes on both sides of the substrate, and the substrate is electrolessly plated on the surface adjacent to and forming the at least one aperture such that conductive metal is deposited on the surface. The plurality of electrodes assists in determining the concentration of the analyte. The conductive metal on the surface establishes an electrical connection between the two sides of the substrate.

### BRIEF DESCRIPTION OF THE DRAWINGS

FIG. 1 is a top perspective view of a sensing instrument or meter according to one embodiment.
FIG. 2 is a top perspective view of the interior of the sensing instrument of FIG. 1.
FIG. 3 is a sensor package according to one embodiment for use with the sensing instrument of FIGs. 1 and 2.
FIG. 4 is a side view of a cartridge containing a plurality of test sensors according to one embodiment.
FIG. 5a is a top view of a substrate with a plurality of apertures formed therein according to one embodiment.
FIG. 5b is a bottom view of the substrate of FIG. 5a.
FIG. 6a is the top view of the substrate of FIG. 5a with a catalytic ink or catalytic polymeric solution applied thereto according to one embodiment.
FIG. 6b is the bottom view of the substrate of FIG. 5b with a catalytic ink or catalytic polymeric solution applied thereto according to one embodiment.
FIG. 6c is an enlarged cross-sectional side view generally taken along line 6c-6c in FIG. 6a.
FIG. 7a is the top view of the substrate of FIG. 6a with a plurality of electrodes formed thereon according to one embodiment.
FIG. 7b is the bottom view of FIG. 6a with an electrical connection formed thereon according to one embodiment.
FIG. 7c is an enlarged cross-sectional side view generally taken along line 7c-7c in FIG. 7a.
FIG. 8 is a top view of the substrate of FIG. 7a with a reagent applied thereto according to one embodiment.
FIG. 9a is a top view of the substrate of FIG. 8 with a lid attached thereto according to one embodiment.
FIG. 9b is a cross-sectional view taken generally along line 9b-9b of FIG. 9a.
FIG. 9c is a cross-sectional view taken generally along line 9c-9c of FIG. 9a.
FIG. 10a is a top view of the substrate of FIG. 8 with a spacer and a lid attached thereto according to one embodiment.
FIG. 10b is a cross-sectional view taken generally along line 10b-10b of FIG. 10a.
FIG. 10c is a cross-sectional view taken generally along line 10c-10c of FIG. 10a.
FIG. 11 a is a top view of the substrate with a plurality of electrodes formed thereon according to one embodiment.
FIG. 11b is a bottom view of the substrate of FIG. 11a with an electrical connection formed thereon according to one embodiment.

### DETAILED DESCRIPTION OF ILLUSTRATED EMBODIMENTS

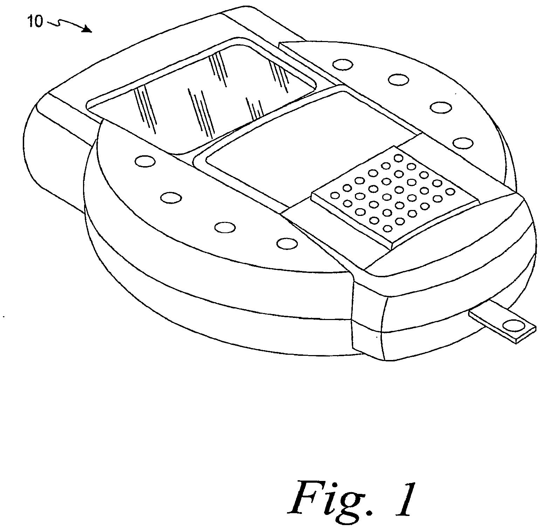

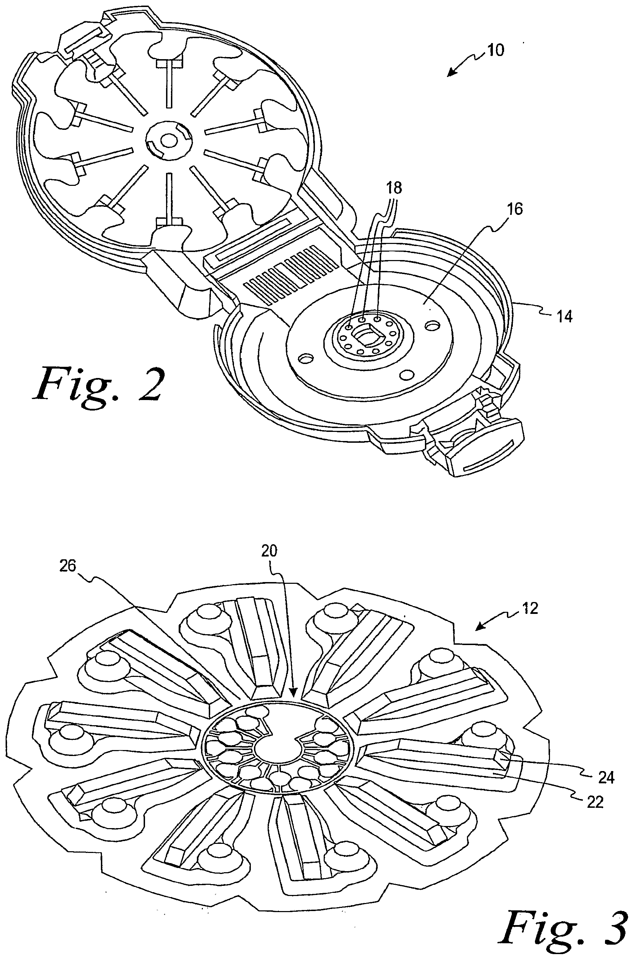

The present invention is directed to a method of forming a test sensor. The test sensors to be formed by the method are adapted to be used in, for example, an instrument or meter 10 such as shown in FIGs. 1-3. In FIG. 2, the inside of the instrument 10 is shown in the absence of a sensor package. One example of a sensor package (sensor package 12) is separately illustrated in FIG. 3. Referring back to FIG. 2, a base member 14 of the instrument 10 supports an auto-calibration plate 16 and a predetermined number of auto-calibration pins 18 that are connected for engagement with the sensor package 12.

The sensor package 12 of FIG. 3 includes an auto-calibration circuit or label 20, a plurality of test sensors 22, and a sensor-package base 26. The plurality of test sensors 22 is used to assist in determining concentrations of analytes. Analytes that may be measured include glucose, lipid profiles (e.g., cholesterol, triglycerides, LDL and HDL), microalbumin, hemoglobin A_{1C}, fructose, lactate, or bilirubin. It is contemplated that other analyte concentrations may be determined. The analytes may be in, for example, a whole blood sample, a blood serum sample, a blood plasma sample, other body fluids like ISF (interstitial fluid) and urine, and non-body fluids. As used within this application, the term "concentration" refers to an analyte concentration, activity (e.g., enzymes and electrolytes), titers (e.g., antibodies), or any other measure concentration used to measure the desired analyte.

In one embodiment, the plurality of test sensors 22 includes an appropriately selected enzyme to react with the desired analyte or analytes to be tested. An enzyme that may be used to react with glucose is glucose oxidase. It is contemplated that other enzymes may be used such as glucose dehydrogenase. It is contemplated that other test sensors may be used.

Referring still to FIG. 3, the plurality of test sensors 22 is stored in individual cavities or blisters 24 and read by associated sensor electronic circuitry before one of the plurality of test sensors 22 is used. In this embodiment, each sensor cavity 24 accommodates one of the plurality of test sensors 22. It is contemplated, however, that the sensor package may be of different shapes then the generally circular shape depicted in FIG. 3. For example, the sensor package may be a square, rectangle, other polygonal shapes, or non-polygonal shapes including oval.

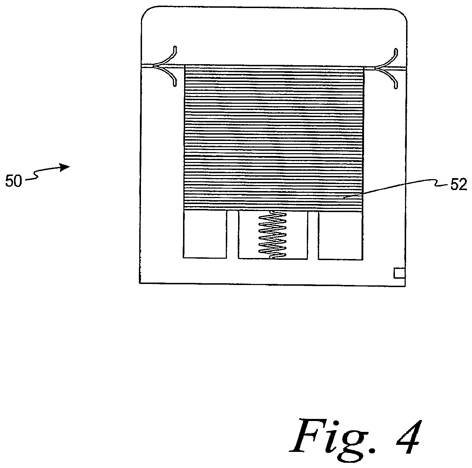

The plurality of test sensors may be stored in, for example, a cartridge such as shown in FIG. 4. FIG. 4 depicts a cartridge 50 that includes a plurality of test sensors 52 that is adapted to be removed one at a time. The cartridge 50 is adapted to be used with an instrument or meter. It is contemplated that the plurality of test sensors may be stored in other cartridges that are adapted to be used with an instrument or meter. It is contemplated that the plurality of test sensors may be stored in a cartridge in which the user manually removes the plurality of test sensors one at a time.

The present invention is directed to the formation of the test sensors. According to one method, a substrate is provided. The substrate forms at least one aperture therethrough. An electroless plating catalyst solution is applied in a pattern on two sides of the substrate. The electroless plating catalyst solution assists in defining the plurality of electrodes and leads on the test sensor. After applying the electroless plating catalyst solution, the substrate is electrolessly plated by an autocatalytic or immersion plating process to form the plurality of the electrodes and leads on the substrate. The plurality of electrodes and leads assist in determining the concentration of the analyte.

The inventive process for forming the test sensors is a process that allows production of the finished plurality of electrodes in a single pass. The single pass avoids between-pass tolerances and associated increase in size and sample volume that happens with multi-pass screen-printing processes. This process thus minimizes registration errors that can occur. The present process is desirably a single-pass process that minimizes any registration errors between a lid and base.

The substrate to be used in the process of forming the test sensors with the plurality of electrodes and leads may be comprised from a variety of materials. The substrate is typically made of insulated material. For example, the substrate may be formed from a polymeric material. Non-limiting examples of polymeric materials that may be used in forming the substrate include polyethylene, polypropylene, oriented polypropylene (OPP), cast polypropylene (CPP), polyethylene terephthlate (PET), polyether ether ketone (PEEK), polyether sulphone (PES), polycarbonate, or combinations thereof.

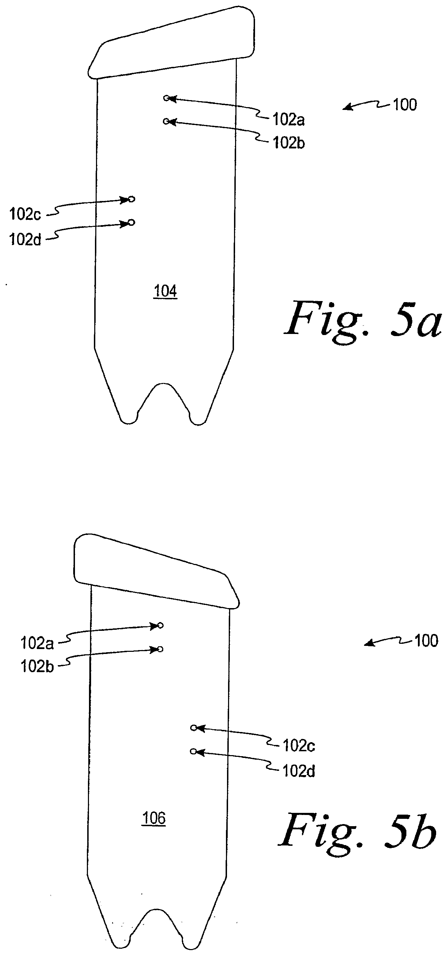

The substrate includes at least one aperture formed therethrough. It is desirable for the substrate to form a plurality of apertures, which in one embodiment may be referred to as via apertures. One non-limiting example of a substrate is shown in FIGs. 5a, 5b. FIGs. 5a, 5b depict a substrate 100 with a plurality of apertures 102a-d formed therein. The diameter of the apertures 102a-d is generally from about 5 to about 30 mils. The periphery of the substrate 100 is roughly rectangular in shape. It is contemplated that the substrate may be of other sizes and shapes.

The plurality of apertures may also be of different shapes and sizes than the generally circular shaped plurality of apertures 102a-d of FIGs. 5a, 5b such as polygonal shapes (e.g., square, rectangle) or non-polygonal shapes (e.g., oval).. The plurality of apertures 102a-d may be formed by a variety of methods including cutting or punching. One method of cutting to form the plurality of apertures 102a-d is by using a laser. As discussed below, the plurality of apertures 102a-d is adapted to connect one of the electrodes with its respective its electrical connection using the two sides 104, 106 of the substrate 100.

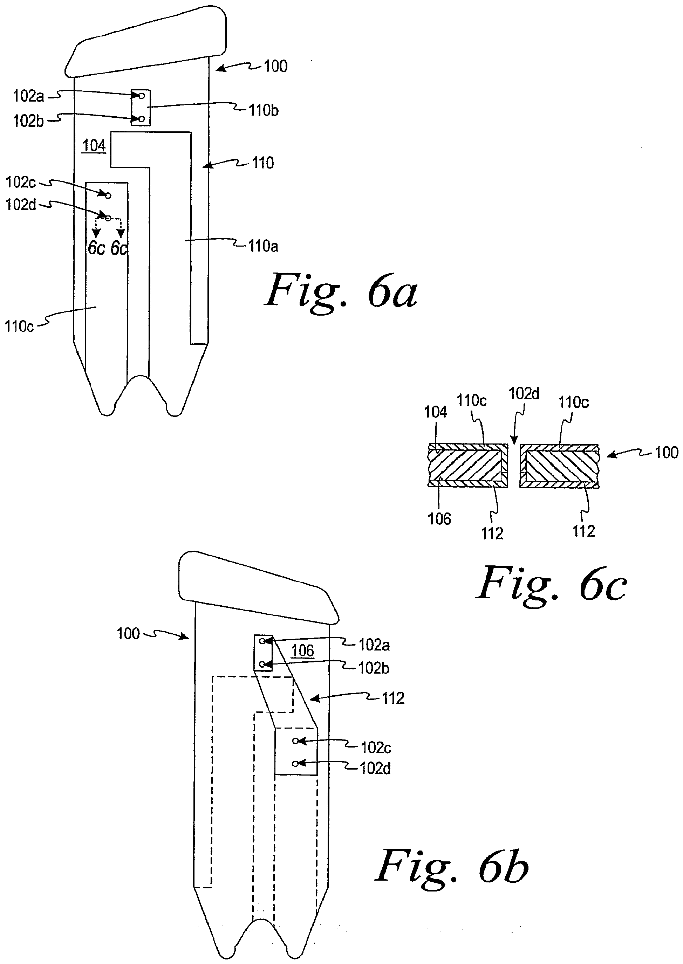

Referring to FIGs. 6a, 6b, the substrate 100 includes a catalytic ink or catalytic polymeric solution 110, 112 thereto. The catalytic ink or catalytic polymeric solution 110a-c is applied to the first side 104 of the substrate 100 while the catalytic ink or catalytic polymeric solution 112 is applied to the second opposing side 106 of the substrate 100. When the catalytic ink or catalytic polymeric solution 110, 112 is applied to the substrate 100, some of the ink or solution 110, 112 is applied onto the inside surfaces forming the plurality of apertures 102a-d. An illustration of the catalytic ink or catalytic polymeric solution 110, 112 applied to the surface adjacent to and forming the aperture 102d is shown in FIG. 6c.

The catalytic ink or catalytic polymeric solution 110a-c is applied in a desired pattern that will eventually form the plurality of electrodes and leads. Specifically, in FIGs. 6a, 6b, the catalytic ink or catalytic polymeric solution 110a-c, 112 is printed in the shape of a plurality of electrodes (e.g., the working electrode and counter electrode) and their electrical connections or leads. It is contemplated that the catalytic ink or catalytic polymeric solution may be applied in other patterns than depicted in FIGs. 6a, 6b.

In one embodiment, the catalytic ink or catalytic polymeric solution 110, 112 applied to the substrate 100 is an ink-jet printable catalytic polymeric solution. The catalytic ink or catalytic polymeric solution adapted to be electrolessly plated may be applied to the substrate by a variety of methods such as screen printing, gravure printing, and ink-jet printing. The catalytic ink or catalytic polymeric solution includes a thermoset or thermoplastic polymer to allow the production of a catalytic film adhered to the substrate. This film is now capable of being electrolessly plated.

According to one method, after the catalytic ink or catalytic polymeric solution is applied, it is dried or cured. One example of a drying or curing process that may be used is curing by ultraviolet light. The drying process may include drying or curing by applying thermal heat. The catalytic ink or catalytic polymeric solution has catalytic properties to allow electroless plating.

After the catalytic ink or catalytic polymeric solution has been applied to the substrate and dried in the process, the substrate is electrolessly plated. Electroless plating uses a redox reaction to deposit conductive metal on the substrate without using an electric current. The conductive metal is generally placed on the predefined pattern of the resulting catalytic film that has been applied to the substrate. Thus, the conductive metal is deposited over the dried or cured catalytic film that includes the electroless plating catalyst.

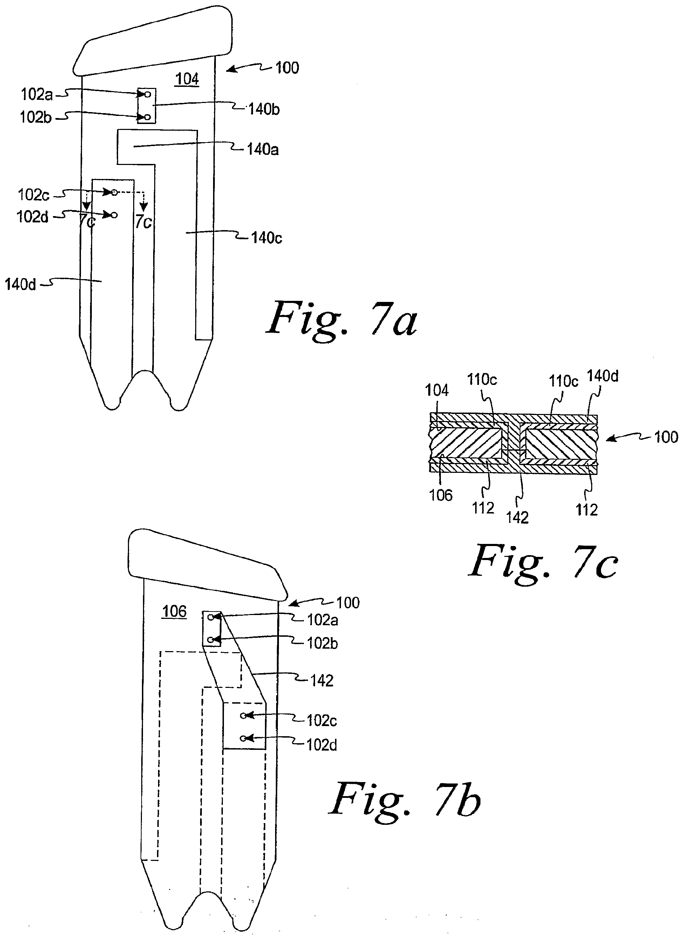

As shown in FIG. 7a, the conductive metal defines the plurality of electrodes 140a, 140b and its respective leads 140c, 140d. The conductive metal also forms the electrical connection 142 between the electrode 140b and its lead 140d. In this embodiment, the electrode 140b is a working electrode and the lead 140d is a working electrode lead. By applying the conductive metal to both sides of the substrate 100 assists in solving the problem of how to precisely define the area of the working electrode with a minimum of printing and handling. This embodiment is also advantageous in that additional space is saved on the first side 104 of the substrate 100 because of the electrical connection 142 being located on the second side 106 of the substrate 100. This additional space may be used to form additional electrodes such as, for example, electrodes adapted to detect underfill or interferants.

The conductive metal located in the plurality of apertures 102a-d establishes the electrical connection between the working electrode 140b and its lead 140d. It is desirable to have a substrate that forms a plurality of apertures in case one of the apertures does not establish an electrical connection.

Non-limiting examples of conductive metals that may be used in electroless plating include copper, nickel, gold, silver, platinum, palladium, rhodium, cobalt, tin, combinations or alloys thereof. For example, a palladium/nickel combination may be used as the conductive metal or a cobalt alloy may be used as the conductive metal. It is contemplated that other metallic materials and alloys of the same may be used in the electroless plating process. It is contemplated that the test sensor may be made from a combination of metals such that a less expensive layer (e.g., nickel or copper) may be plated first and then an enzyme catalyzible metal (e.g., gold, platinum, palladium or rhodium) may be added later. The thickness of the conductive metallic material may vary, but generally is from about 0.025 µm to about 2.54 µm (1 to about 100 µ inches) and, more typically, from about 0.127 µm to about 1.27 µm (5 to about 50 µ inches).

The electroless plating process typically involved reducing a complex metal in an aqueous solution. The substrate may be electrolessly plated by an autocatalytic or immersion plating process. The aqueous solution typically includes a mild or strong reducing agent that varies by the metal or the bath. One reducing agent that may be used in electroless plating is sodium hypophosphite (NaH₂PO₂). It is contemplated that other reducing agents may be used in electroless plating. The aqueous solution may be located in a container, which is referred to as an electroless plating bath. Thus, in one process, the substrate 100 proceeds through an electroless plating bath containing the conductive metal that forms the plurality of electrodes and leads.

The substrate is removed from the bath and is dried to form the plurality of electrodes, leads and electrical connections. Specifically, the conductive metal located in the plurality of apertures establishes the electrical connection between the sides of the substrate. This is illustrated, for example, in FIG. 7c where a plating layer (which forms the lead 140d and the electrical connection 142) is formed on the catalytic ink or catalytic polymeric solution 110c, 112 and also extends into the aperture. The plating layer may substantially fill the aperture such as shown in FIG. 7c. The plating layer needs to be in a sufficient quantity and properly located in the aperture so as to establish an electrical connection between the sides 104, 106 of the substrate 100.

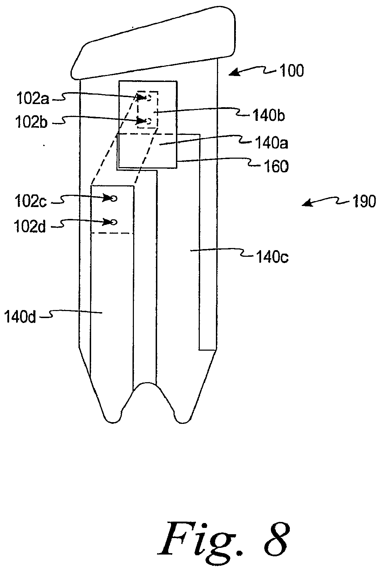

The electrodes, leads and electrical connections are dried and then a reagent layer is applied. For example, as shown in FIG. 8, a test sensor 190 includes the substrate 100 and an area 160 that includes an enzyme to assist in determining the analyte concentration of the fluid sample. Specifically, a fluid sample contacts the area 160 with the enzyme. The area 160 may include other materials to assist in determining the analyte concentration of the fluid sample.

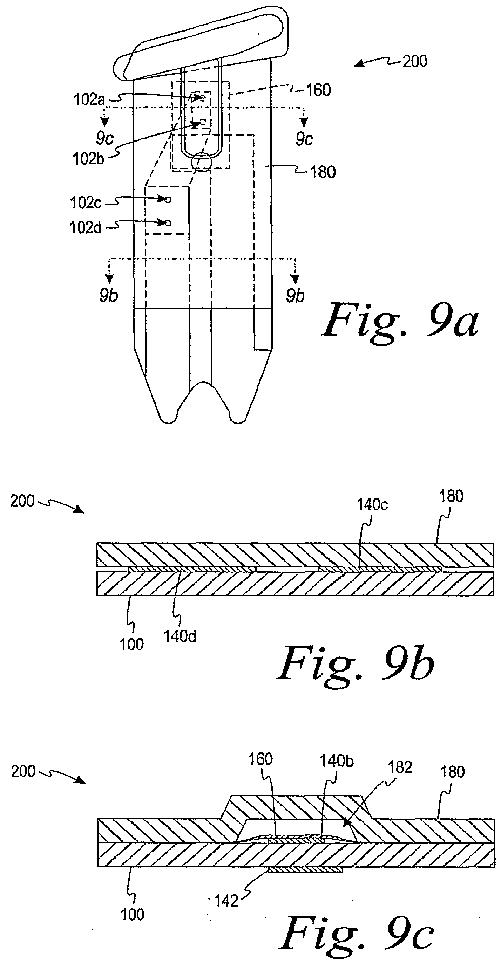

Referring to FIG. 9a, a test sensor 200 that assists in determining the analyte according to one embodiment is depicted. The test sensor 200 is the same as the test sensor 190 of FIG. 8, but with a lid 180 being added that is attached to the substrate 100. The substrate 100 and the lid 180 may be attached by a variety of methods include heat-sealing and by using an adhesive. FIGs. 9b, 9c depict various cross-sectional views of the test sensor 200. FIG. 9c includes a space 182 formed between the lid 180 and substrate 100 with the electrode 140b and the reagent layer 160.

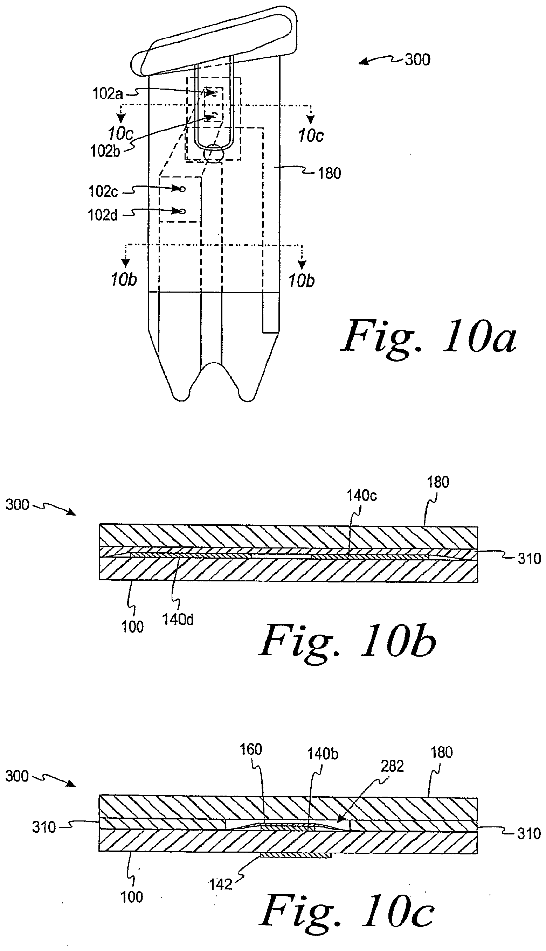

In another embodiment, a spacer and lid are added to the substrate to form a test sensor. Referring to FIGs. 10a, 10b, a test sensor 300 is the same as the test sensor 190 of FIG. 8, but with a spacer 310 and the lid 180 being added. The spacer 310 and the lid 180 may be added together or separately to the substrate 100. FIGs. 10b, 10c depict various cross-sectional views of the test sensor 300. FIG. 10c includes a space 282 formed between the lid 180, substrate 100 and located between the spacer 310.

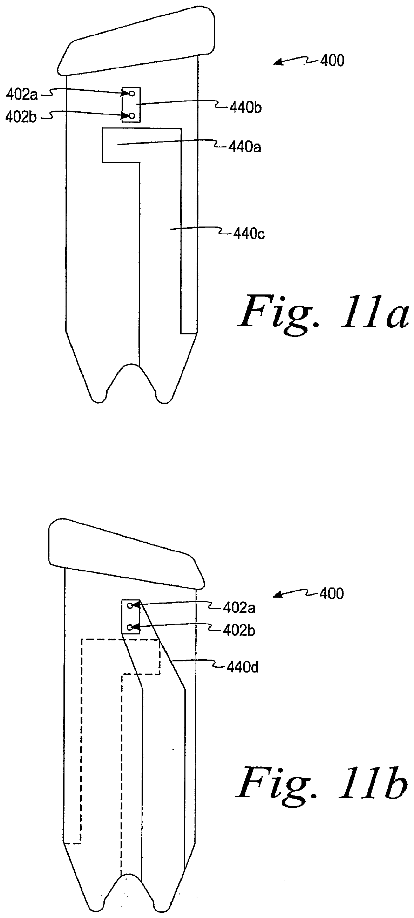

It is also contemplated in another embodiment that the electrode lead of one of the electrodes may be entirely located on a first side of the substrate while the electrode lead of another electrode may be entirely located on a second side of the substrate. For example, referring to FIGs. 11 a, 11b, a substrate 400 is depicted. The substrate 400 includes an electrode lead 440c and an electrode lead 440d, which are located on opposing sides of the substrate 400. In this embodiment, the substrate 400 includes a plurality of electrodes 440a, 440b and an electrical connection is established via the apertures 402a, 402b formed in the substrate 400. In one embodiment, the electrode 440a is a counter electrode and the electrode 440b is a working electrode. In this embodiment, the electrode 440b is electrically connected to its lead 440d via the apertures 402a, 402b.
applying a catalytic ink or catalytic polymeric solution in a pattern on two sides of the substrate, the catalytic ink or catalytic polymeric solution assisting in defining the plurality of electrodes on the test sensor; and
after applying the catalytic ink or catalytic polymeric solution, electroless plating of the substrate to form the plurality of the electrodes of the substrate, the plurality of electrodes assisting in determining the concentration of the analyte.

### PROCESS B

The method of process A wherein the substrate is a polymeric material.

### PROCESS C

The method of process B wherein the polymeric material includes polyethylene, polypropylene, oriented polypropylene (OPP), cast polypropylene (CPP), polyethylene terephthlate (PET), polyether ether ketone (PEEK), polyether sulphone (PES), polycarbonate, or combinations thereof.

### PROCESS D

The method of process A wherein the electroless plating uses a conductive metal being copper, nickel, gold, silver, platinum, palladium, rhodium, cobalt, tin, combinations or alloys thereof.

### PROCESS E

The method of process D wherein the thickness of the conductive metallic material is from about 1 to about 100 µ inches.

### PROCESS F

The method of process E wherein the thickness of the conductive metallic material is from 5 to about 50 µ inches.

### PROCESS G

The method of process A wherein the catalytic ink or catalytic polymeric solution is applied onto the substrate by ink-jet printing.

### PROCESS H

The method of process A wherein the catalytic ink or catalytic polymeric solution is applied onto the substrate by screen printing.

### PROCESS I

The method of process A wherein the catalytic ink or catalytic polymeric solution is applied onto the substrate by gravure printing.

### PROCESS J

The method of process A further including drying or curing the catalytic ink or catalytic polymeric solution.

### PROCESS K

The method of process A wherein the at least one aperture is a plurality of apertures.

### PROCESS L

The method of process K wherein the plurality of apertures is formed by a laser prior to defining the plurality of electrodes on the substrate.

### PROCESS M

The method of process K wherein the plurality of apertures is formed by punching prior to defining the plurality of electrodes on the substrate.

### PROCESS N

The method of process A further including the act of attaching a lid to the substrate.

### PROCESS O

The method of process A further including the acts of providing a lid, attaching a spacer to the substrate, the spacer being located between the lid and the substrate.

### PROCESS P

The method of process A further applying an enzyme to the substrate.

### PROCESS Q

The method of process P wherein the enzyme is glucose oxidase or glucose dehydrogenase.

### PROCESS R

A method of forming a plurality of electrodes on a test sensor, the test sensor assisting in determining the concentration of an analyte, the method comprising the acts of:
providing a substrate;
forming a plurality of apertures through the substrate;
applying a catalytic ink or catalytic polymeric solution in a pattern on two sides of the substrate, the catalytic ink or catalytic polymeric solution assisting in defining the plurality of electrodes on the test sensor;
after applying the catalytic ink or catalytic polymeric solution, electroless plating of the substrate with a conductive metal to form the plurality of the electrodes of the substrate, the plurality of electrodes assisting in determining the concentration of the analyte; and
further applying an enzyme to the substrate.

### PROCESS S

The method of process R wherein the substrate is a polymeric material.

### PROCESS T

The method of process R wherein the electroless plating uses a conductive metal being copper, nickel, gold, silver, platinum, palladium, rhodium, cobalt, tin, combinations or alloys thereof.

### PROCESS U

The method of process R wherein the catalytic ink or catalytic polymeric solution is applied onto the substrate by screen printing.

### PROCESS V

The method of process R wherein the catalytic ink or catalytic polymeric solution is applied onto the substrate by gravure printing.

### PROCESS W

The method of process R wherein the catalytic ink or catalytic polymeric solution is applied onto the substrate by ink-jet printing.

### PROCESS X

The method of process R wherein the at least one aperture is a plurality of apertures.

### PROCESS Y

The method of process X wherein the plurality of apertures is formed by a laser prior to defining the plurality of electrodes on the substrate.

### PROCESS Z

The method of process X wherein the plurality of apertures is formed by punching prior to defining the plurality of electrodes on the substrate.

### PROCESS AA

The method of process R further including drying or curing the catalytic ink or catalytic polymeric solution.

### PROCESS BB

The method of process R further including the act of attaching a lid to the substrate.

### PROCESS CC

The method of process R further including the acts of providing a lid, attaching a spacer to the substrate, the spacer being located between the lid and the substrate.

### PROCESS DD

The method of process R wherein the enzyme is glucose oxidase or glucose dehydrogenase.

## Claims

1. A method of forming a plurality of electrodes on a test sensor, the test sensor assisting in determining the concentration of an analyte, the method comprising the acts of:
providing a substrate;
forming at least one aperture through the substrate;
applying a catalytic ink or catalytic polymeric solution in a pattern on two sides of the substrate and on a surface adjacent to and forming the at least one aperture, the catalytic ink or catalytic polymeric solution on the two sides assisting in defining the plurality of electrodes on the test sensor; and
after applying the catalytic ink or catalytic polymeric solution, electroless plating of the substrate to form the plurality of the electrodes on both sides of the substrate and electroless plating on the surface adjacent to and forming the at least one aperture such that conductive metal is deposited on the surface, the plurality of electrodes assisting in determining the concentration of the analyte and the conductive metal on the surface establishing an electrical connection between the two sides of the substrate.

2. The method of claim 1, wherein the electroless plating uses a conductive metal being copper, nickel, gold, silver, platinum, palladium, rhodium, cobalt, tin, combinations or alloys thereof.

3. The method of claim 2, wherein the thickness of the conductive metallic material is from about 0.025 µm to about 2.54 µm (1 to about 100 µ inches).

4. The method of claim 3, wherein the thickness of the conductive metallic material is from 0.127 µm to about 1.27 µm (5 to about 50 µ inches).

5. The method of claim 1, wherein the catalytic ink or catalytic polymeric solution is applied onto the substrate by ink-jet printing.

6. The method of claim 1, wherein the catalytic ink or catalytic polymeric solution is applied onto the substrate by screen printing.

7. The method of claim 1, wherein the catalytic ink or catalytic polymeric solution is applied onto the substrate by gravure printing.

8. The method of claim 1, wherein the at least one aperture is a plurality of apertures, the plurality of apertures being formed by a laser prior to defining the plurality of electrodes on the substrate.

9. The method of claim 1, wherein the at least one aperture is a plurality of apertures, the plurality of apertures being formed by punching prior to defining the plurality of electrodes on the substrate.

10. The method of claim 1 further including the act of attaching a lid to the substrate.

11. The method of claim 1 further including the acts of providing a lid, attaching a spacer to the substrate, the spacer being located between the lid and the substrate.

12. The method of claim 1 further including the act of applying an enzyme to the substrate.

13. The method of claim 1, wherein the enzyme is glucose oxidase or glucose dehydrogenase.

## Patentansprüche

1. Verfahren zum Bilden einer Anzahl von Elektroden an einem Testsensor, wobei der Testsensor beim Bestimmen der Konzentration eines Analyten mitwirkt, wobei das Verfahren die folgenden Schritte umfasst:
Bereitstellen eines Substrats;
Bilden wenigstens einer Öffnung durch das Substrat hindurch;
Anwenden einer katalytischen Tinte oder katalytischen polymeren Lösung in einem Muster auf zwei Seiten des Substrats und auf einer Oberfläche,
die zu der wenigstens einen Öffnung benachbart angeordnet ist und diese bildet, wobei die katalytische Tinte oder katalytische polymere Lösung auf den beiden Seiten ein Bilden der Anzahl von Elektroden an dem Testsensor unterstützt; und
nach dem Anwenden der katalytischen Tinte oder katalytischen polymeren Lösung eine stromlose Abscheiden des Substrats, um die Anzahl von Elektroden zu beiden Seiten des Substrats zu bilden, und ein stromloses Abscheiden auf der Oberfläche, die zu der wenigstens einen Öffnung benachbart angeordnet ist und diese bildet, so dass auf der Oberfläche leitendes Metall abgeschieden wird, wobei die Anzahl von Elektroden ein Bestimmen der Konzentration des Analyten unterstützt, und das leitende Metall auf der Oberfläche eine elektrische Verbindung zwischen beiden Seiten des Substrats errichtet.

2. Verfahren nach Anspruch 1, wobei das stromlose Abscheiden ein leitendes Metall verwendet, das auf Kupfer, Nickel, Gold, Silber, Platin, Palladium, Rhodium, Kobalt, Zinn, einer Kombination von diesen oder auf deren Legierungen basiert.

3. Verfahren nach Anspruch 2, wobei die Dicke des leitenden metallischen Materials in einem Bereich von etwa 0,025 µm bis etwa 2,54 µm (1 bis etwa 100 µ-Zoll) liegt.

4. Verfahren nach Anspruch 3, wobei die Dicke des leitenden metallischen Materials von 0,127 µm bis etwa 1,27 µm (5 bis etwa 50 µ-Zoll) beträgt.

5. Verfahren nach Anspruch 1, wobei die katalytische Tinte oder katalytische polymere Lösung durch Tintenstrahldruck auf das Substrat aufgetragen wird.

6. Verfahren nach Anspruch 1, wobei die katalytische Tinte oder katalytische polymere Lösung durch Siebdruck auf das Substrat aufgetragen wird.

7. Verfahren nach Anspruch 1, wobei die katalytische Tinte oder katalytische polymere Lösung durch Tiefdruck auf das Substrat aufgetragen wird.

8. Verfahren nach Anspruch 1, wobei die wenigstens eine Öffnung mehrere Öffnungen umfasst, wobei die mehreren Öffnungen durch einen Laser ausgebildet werden, bevor die Anzahl von Elektroden auf dem Substrat gebildet werden.

9. Verfahren nach Anspruch 1, wobei die wenigstens eine Öffnung mehrere Öffnungen umfasst, wobei die mehreren Öffnungen durch Stanzen ausgebildet werden, bevor die Anzahl von Elektroden auf dem Substrat gebildet werden.

10. Verfahren nach Anspruch 1, ferner mit dem Schritt eines Anbringens eines Deckels an dem Substrat.

11. Verfahren nach Anspruch 1, ferner mit den Schritten des Bereitstellens eines Deckels und Anbringens einer Abstandsschicht an dem Substrat, wobei die Abstandsschicht zwischen dem Deckel und dem Substrat angeordnet ist.

12. Verfahren nach Anspruch 1, ferner mit dem Schritt eines Auftragens eines Enzyms auf dem Substrat.

13. Verfahren nach Anspruch 1, wobei das Enzym Glucoseoxidase oder Glucosedehydrogenase ist.

## Revendications

1. Procédé de formation d'une série d'électrodes sur un capteur de test, le capteur de test contribuant à la détermination de la concentration d'un analyte, le procédé comprenant les étapes consistant à :
préparer un substrat ;
former au moins une ouverture dans le substrat ;
appliquer une encre catalytique ou une solution polymère catalytique selon un motif sur deux faces du substrat et sur une surface adjacente et former au moins une ouverture, l'encre catalytique ou la solution polymère catalytique sur les deux faces contribuant à définir la série d'électrodes sur le capteur de test, et
après application de l'encre catalytique ou de la solution polymère catalytique, dépôt autocatalytique sur le substrat pour former la série des électrodes sur les deux faces du substrat et dépôt autocatalytique sur la surface adjacente et formation d'au moins une ouverture de sorte qu'un métal conducteur est déposé sur la surface, la série d'électrodes contribuant à déterminer la concentration de l'analyte et le métal conducteur sur la surface établissant une connexion électrique entre les deux faces du substrat.

2. Procédé selon la revendication 1, où le dépôt autocatalytique utilise un métal conducteur, qui est le cuivre, le nickel, l'or, l'argent, le platine, le palladium, le rhodium, le cobalt, l'étain, leurs combinaisons ou alliages.

3. Procédé selon la revendication 2, où l'épaisseur du matériau métallique conducteur se situe dans l'intervalle allant de 0,025 µm à environ 2,54 µm (1 à environ 100 µpouces).

4. Procédé selon la revendication 3, où l'épaisseur du matériau métallique conducteur se situe dans l'intervalle allant de 0,127 µm à environ 1,27 µm (5 à environ 50 µpouces).

5. Procédé selon la revendication 1, où l'encre catalytique ou la solution polymère catalytique est appliquée sur le substrat par impression par jet d'encre.

6. Procédé selon la revendication 1, où l'encre catalytique ou la solution polymère catalytique est appliquée sur le substrat par sérigraphie.

7. Procédé selon la revendication 1, où l'encre catalytique ou la solution polymère catalytique est appliquée sur le substrat par héliogravure.

8. Procédé selon la revendication 1, où là au moins une ouverture est une série d'ouvertures, la série d'ouvertures étant formées par un laser avant de définir la série des électrodes sur le substrat.

9. Procédé selon la revendication 1, où là au moins une ouverture est une série d'ouvertures, la série d'ouvertures étant formées par emboutissage avant de définir la série des électrodes sur le substrat.

10. Procédé selon la revendication 1, comprenant en outre, l'étape de fixation d'un couvercle sur le substrat.

11. Procédé selon la revendication 1, comprenant en outre, l'étape de fixation d'un couvercle, et d'un écarteur au substrat, l'écarteur se trouvant entre le couvercle et le substrat.

12. Procédé selon la revendication 1, comprenant en outre, l'étape d'application une enzyme sur le substrat.

13. Procédé selon la revendication 1, où l'enzyme est la glucose oxydase ou la glucose déshydrogénase.
